Europäisches Patentamt

(19) European Patent Office

Office européen des brevets

(11) Publication number: **0 206 561 B1**

(12) **EUROPEAN PATENT SPECIFICATION**

(45) Date of publication of patent specification:
30.01.91 Bulletin 91/05

(51) Int. Cl.⁵: **G01N 33/53**, G01N 33/543, G01N 33/52, C12Q 1/68

(21) Application number: 86304147.1

(22) Date of filing: 30.05.86

(54) Diagnostic device.

The file contains technical information submitted after the application was filed and not included in this specification

(30) Priority: 31.05.85 US 740100
05.05.86 US 857914

(43) Date of publication of application:
30.12.86 Bulletin 86/52

(45) Publication of the grant of the patent:
30.01.91 Bulletin 91/05

(84) Designated Contracting States:
AT BE CH DE FR GB IT LI LU NL SE

(56) References cited:
EP-A- 0 097 952
EP-A- 0 159 727
EP-A- 0 176 792
US-A- 3 888 629
US-A- 4 256 693

(56) References cited:
CLINICAL CHEMISTRY, vol. 31, no. 9, September 1985, pages 1427-1431, Winston-Salem, North Carolina, US; G.E. VALKIRS et al.: "Immunoconcentration - a new format for solid-phase immunoassays" ANALYTICAL CHEMISTRY, vol. 55, no. 4, April 1983, pages 498-514, Easton, Pennsylvania, US; B. WALTER "Dry reagent chemistries in clinical analysis"

(73) Proprietor: Murex Corporation
P O Box 2003 3000 Northwoods Parkway
Norcross, GA 30091 (US)

(72) Inventor: Hossom, Miles Gerald
3585 Schilling Ridge N.W.Duluth
Georgia 30136 (US)
Inventor: Jacob, Dinesh Andrew
1 Thornton Close
Girton Cambridge CB3 0NF (GB)

(74) Representative: Perry, Robert Edward et al
GILL JENNINGS & EVERY 53-64 Chancery
Lane
London WC2A 1HN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

## BACKGROUND OF THE INVENTION

The present invention relates to diagnostic devices for analyte assay. More particularly it relates to devices and methods utilizing filter means for testing biological fluids to detect the presence of analytes such as bacterial, viral, parasitic, or fungal antigens and immunoglobulins, hormones, serum proteins, drugs, and the like.

The present invention also relates to an improved diagnostic device for analyte assay which includes apparatus for the separation of solid phase materials from liquid phase materials. More particularly, the present invention comprises an improved diagnostic device which includes a container for combining fluid and/or solid materials in a convenient, inexpensive manner which can be used to effectively deliver the materials, either prior to, during, or subsequent to a reaction, to a filter means in the improved diagnostic device.

At the present time there are a number of devices and procedures disclosed for diagnosing the presence of such analytes by means of reaction occurring on filters but they are either too complex, costly, inaccurate, time-consuming or a combination of such factors.

For example, U.S. Patent 3,888,629 discloses a reaction cell having a matrix pad for carrying out immunoassays. The pad serves as the means for retaining the reagents and as the site in which the reaction occurs as one or more of the fluid reagents are added to the pad and pass therethrough to the absorbent material directly below. In addition to the many time-consuming steps required to process the pad to determine the results of the test; including removing it from the device, such device is essentially limited to isotopic tests. It is not practical for nonisotopic tests such as enzyme-linked immunoassays, since the device requires removal of the absorbent pad for viewing. Moreover, certain biological fluids, such as blood sera, contain particulate and/or colored matter which tend to remain on the surface of the matrix pad and thus make it difficult, if not impossible, to obtain an accurate reading in nonisotopic immunoassay procedures. Further, by passing the reagents directly through the entire surface area of the matrix pad there is often poor separation of the analyte since the absorbent pad is very thin (thereby affording only a very short distance for separation to occur) and there is limited concentration of analyte at any location on or in the pad.

Efforts to improve such device are reflected in US-A-4246339 and US-A-4407943 which try to limit the area of the fibre through which one or more of the reagents must pass. Here again, however, there is flow directly through the thin filter to the absorbent material below the filter, again resulting in poor separation and difficulty in obtaining accurate readings when specimens are being tested which contain particulate and/or coloured matter which is retained on the surface of the filter.

EP-A-0097952 discloses a multilayer analytical element for fluorimetric assay of an analyte, comprising a laminated structure, of layers in the following order :

(a) a porous sheet containing a predetermined amount of fluorescence-labelled analyte ;

(b) a porous separation sheet ; and

(c) a reaction sheet containing, in fixed form, a predetermined amount of protein specifically bindable to the analyte.

Another multilayered analytical element is disclosed in US-A-4256693, for use in the analysis of blood. It comprises a laminated structure, of layers in the following order :

(a) a filter layer capable of removing formed components from blood ;

(b) a water-proof layer with at least one small opening ;

(c) a porous spreading layer ; and

(d) a reagent layer.

It is an object behind the present invention to obviate the problems associated with the known devices, and to provide a device for rapid and accurate analyte assay.

## Summary of the Invention

According to the present invention, a device for testing for the presence of an analyte in a liquid, comprises

an aperture through which the liquid can be introduced ;

a filter comprising a layer of porous material capable of drawing liquid through its structure by capillary action and which includes a reaction zone which receives liquid through the aperture, and which functions to separate the analyte (if present) from the liquid ;

absorbent means and means for retaining the filter in position beneath the aperture ;

characterised in that the filter includes a peripheral zone and a reaction zone which is adapted to be viewable, and the absorbent means is associated with the peripheral zone, but not the reaction zone, of the filter, whereby liquid is drawn from the reaction zone to the peripheral zone.

The present invention provides an improved diagnostic device and can comprise a substantially cylindrical body portion and a removable cap which has a liquid inlet extending into the cylindrical body portion and includes a conical shaped portion leading to a liquid discharge aperture formed therein. A pre-filter may be formed either in the liquid inlet or in a pre-filter container which can be used with the device. The pre-filter container can have a substantially cylindrical body which conforms to and can be inserted in the liquid inlet forming part of the removable cap. The container has an open upper end and an associated removable closure, an open bottom end sealed with a frangible material and, if desired, a pre-filter positioned in the container between the upper and lower ends. When liquid is placed in the container, the container may be inserted in the liquid inlet forming part of the cap for the diagnostic device ; a puncture device associated with the liquid inlet ruptures the frangible material sealing the lower end, thereby allowing the liquid to be funnelled to a reaction zone on a filter placed beneath or below the discharge aperture. Discharge apertures of various sizes can be formed in the removable caps, thereby allowing a particular cap to be associated with a device for a particular test.

The novel device preferably includes a reaction reservoir container for removable insertion in the liquid input means receiving inlet, and a support shoulder extending radially and circumferentially about the exterior of the container for resting on the liquid input means when the container is inserted in the receiving inlet, thereby holding the container in a secure relationship in the receiving inlet.

## Brief Description of the Drawings

The present invention will now be disclosed in conjunction with the accompanying drawings, in which like numerals represent like components, and in which :

Fig. 1 is a cross-sectional view of a test device of the present invention ;

Fig. 2 is an exploded perspective cross-sectional view of the test device ;

Fig. 3 is a bottom view of the dispensing means and a portion of the casing means ;

Fig. 4 is a cross-sectional view of an alternative embodiment of the test device having absorbent material above and below the filter means ;

Fig. 5 is an alternative embodiment of the invention having an absorbent means below the filter means ;

Fig. 6 is an alternative embodiment of the test device having a rectangular shape ;

Fig. 7 is another alternative embodiment having absorbent material on either side of the liquid dispensing means ;

Fig. 8 is yet another alternative embodiment having liquid dispensing means positioned at one end of the device and absorbent material at the remaining space ;

Fig. 9 is an embodiment having liquid dispensing means shaped as a trough containing a plurality of discrete liquid discharge apertures contacting filter means defining a plurality of reaction zones ;

Fig. 10 is an embodiment showing a combination of the individual devices joined together ;

Fig. 11 is an isometric view of a premixing container which can be used with the test device shown in Fig. 1 or Fig. 13 or Fig. 14 ;

Fig. 12 is an isometric view of a device for puncturing the frangible membrane on the bottom of the premixing container shown in Fig. 11 ;

Fig. 13 is a cross-sectional view of the test device of the present invention with the premixing container shown in Fig. 11 inserted in the liquid receiving inlet and with the rupturing device shown in Fig. 12 in the bottom portion thereof with the point downward so that the device can be shipped or stored as an integrated unit ;

Fig. 14 is a cross-sectional view of the test device of the present invention with the rupturing device of Fig. 12 inserted in the bottom of the liquid input means in a manner in which the points are upward and in which the premixing container shown in Fig. 11 has been inserted in the liquid input receiving means and the frangible membrane on the bottom thereof ruptured by the punctured means thereby allowing any fluid therein to be funneled onto the filter of the test device ;

Fig. 15 is a cross-sectional view of the ap for an improved diagnostic device which includes the liquid input receiving inlet and the discharge aperture ;

Fig. 16 is a cross-sectional view of the cylindrical body portion of the improved diagnostic device with the retainer portion holding the filter and absorbent means in place in the body portion ;

Fig. 17 is an isometric cross-sectional view of the cap illustrating the liquid input means and the liquid discharge aperture ;

Fig. 18 is a cross-sectional isometric view of the body portion with the retainer portion holding the filter and absorbent means in place and illustrating threads on the upper portion of the cylindrical body portion to which the cap in Figs. 15 and 17 can be removably secured with mating threads ;

Fig. 19 is a bottom view of the closure or retainer means illustrating the center viewing port as well as two additional ports spaced from the center viewing port for the purpose of detecting when improper washing has occurred during the testing ; and

Fig. 20 is a bottom view of the closure or retainer means illustrating three ports spaced from the center viewing port, two of which indicate whether or not proper washing has occurred and the third of which represents a control test point which confirms the reaction that takes place in the center viewing port.

## DETAILED DESCRIPTION OF THE DRAWINGS

The device of the present invention is suitable for use with any of the conventional procedures used for analyte assays such as isotopic assays and nonisotopic assays such as competitive or non-competitive enzyme-linked immunoassays, enzyme multiplied immunoassays, enzyme inhibition immunoassays, heterogeneous or homogeneous fluorescent immunoassays, chemiluminescent and bioluminescent assays, those assays using labeled RNA or DNA probes, and the like.

The particular analyte assay test to be used will depend upon the particular analyte and the desire of the person carrying out the test. The only essential requirement for each particular test used is that the device of the instant invention be structured, as discussed below, so as to insure that all fluids and reactants necessary to carry out the test are caused to flow outwardly through the filter means from the point of application onto a localized portion of the top surface of the filter to peripheral portions in the filter and that no fluid passes completely through the filter at the point of application. This critical aspect of this invention results in better separation of analyte since it travels further transversely through the filter means, concentration of all the analyte and other reactants at a localized portion of the filter means resulting in more accurate results, and permits top, bottom, and straight-through reading of the filter means to determine the results of the test.

Other than this requirement of the present invention, all of the other steps, conditions, reactants, and the like of the various conventional analyte assays set forth above are those conventionally used in such procedures.

The invention can be more fully understood with reference to the drawings in which like numerals represent like elements.

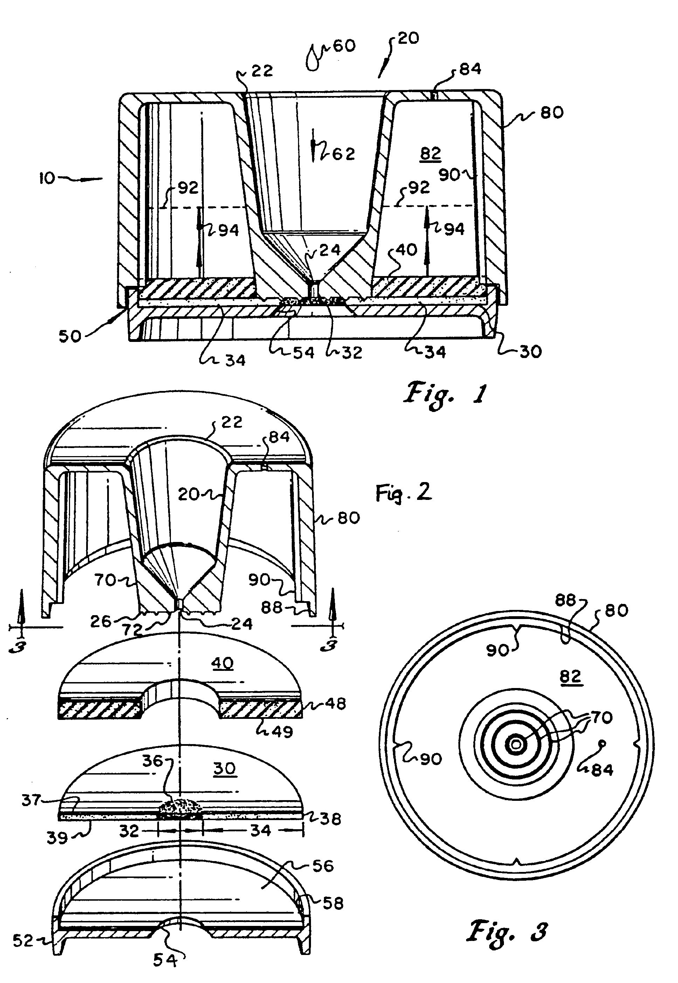

The basic operation of the inventive test device can be understood with reference to **Fig. 1** in which the test device **10** comprises a liquid input means **20** with a filter means **30** positioned below said input means **20** having a reaction zone **32** for receiving the liquid from said liquid input means **20** and a peripheral zone **34** associated with the reaction zone **32**. Associated with only the peripheral zone **34** of filter means **30** is an absorbent means **40**. Further, there is a retainer means **50** comprised of closure means **52** for holding the filter means **30** in position below the input means **20** such that the reaction zone **32** receives liquid **60** from said liquid input means **20** through discharger aperture **24**. Thus, it can be understood that a liquid **60** placed or poured into receiving inlet **22** of input means **20** will flow through discharge aperture **24** into reaction zone **32** of filter means **30**. Liquid **60** passes through filter means **30** transversely from reaction zone **32** into peripheral zone **34**. A reaction, such as filter separation or immunological binding, may take place in the reaction zone **32** as the liquid is diffused therethrough and color changes or other reaction reading signals produced in the reaction zone **32** can be viewed or read through viewing port **54** in closure means **52**. The unreacted liquid is absorbed by absorbent means **40** which is in contact only with the peripheral zone **34** without directly contacting reaction zone **32**. Thus, the liquid passes through reaction zone **32** before being absorbed. The absorbent means **40** is positioned within hollow cavity **82** formed within casing means **80** which is removably or permanently press-fit onto closure means **52**. As liquid is absorbed by absorbent means **40**, air within hollow cavity **82** is displaced by the liquid. The displaced air escapes through vent **84** so that the air pressure is equalized. All components other than the filter means **30** and the absorbent means **40**, the construction of which will be described below, may be formed of any suitable inert material such as molded polystyrene or other plastic materials. The material is preferably opaque and preferably white in color so that color interference with the reaction signal is reduced. In addition, while the shape of casing means **80** is shown to be substantially cylindrical, it may have other shapes such as square, octagon, and the like.

The amount of liquid used depends upon the test, assay or immunoassay being performed. In any given test more than one type of liquid may be used in a predetermined sequence. For example, a fluid may be used to prepare the reaction zone, then a washing fluid or solvent added, then a bodily fluid specimen and then another washing liquid added, then a reaction indicator fluid or coloring agent added and then another washing fluid added. The capacity of the absorbent means must be sufficient to handle all the liquid used in the test. The large volume of the inventive device allows the user added flexibility in the tests which can be performed. Also an inert reaction zone **32** can be used because the device has sufficient capacity for preparatory additions of liquid to form an active reaction zone.

The liquid input **60** into the liquid input means flows in liquid flow direction **62**. It will be understood that in the preferred embodiment the motivating force of the flow is gravity such that the liquid flows through liquid input means **20** generally from top to bottom or from receiving inlet **22** to discharge aperture **24**.

The liquid flows through discharge aperture **24** onto filter means **30** positioned below the input means **20**. Filter means **30** has at least one reaction zone **32** for receiving liquid from said input means. Also filter means **30** has at least one peripheral zone **34** associated with the reaction zone **32**. The diameter of discharge aperture **24** is sufficient in connection with the liquid pressure head present in input means **20** so that the liquid will discharge onto upper surface **37** of filter means **30** and will not be forced straight through the filter **30** and out the bottom surface **39**. Thus, the hydrostatic pressure is adjusted so that the liquid enters the filter by gravitational forces and it is diffused through the filter **30** by capillary action. It has been found that, for an input funnel **20** which is approximately 1.2 inches high and a filter **30** which is 0.03 inch thick, a discharge aperture **24** having an approximate diameter of 0.06 inch is sufficient. Absorbent means **40** acts to ensure the outward fluid flow so that no liquid goes completely through filter means **30** at the point of application from input means **20**. Thus, the diameter and height of the discharge aperture **24**, type and thickness of filter means **30**, and type and thickness of absorbent means **40** are correlated to ensure that no liquid used for any particular assay will be passed straight through the filter, but will travel transversely outwardly from the point of application in the filter plane. The particular dimensions for each assay procedure can be readily determined by routine experimentation.

A unique aspect of the present invention is the combination of the pushing force of gravity and the outward transverse pulling force of the capillary action of the filter means **30** and absorbent means **40**. Other devices commercially available make use of predominantly either only a pushing force or a pulling force. Examples of such devices are those that drop compounds onto the filter via a pipette, relying on a "radial" flow of fluid through the filter, or a reservoir means whereby a column of fluid is drawn straight through the filter without any transverse flow of fluid along the plane of the filter. The present invention employs both forces so that a column of fluid is pushed onto the filter by hydrostatic pressure, and subsequently drawn by capillary action outward through the filter from the point of application to a peripheral zone and then into the absorbent means. The use of these motivating forces effects a more rapid and complete filtration and separation of components in the filter means.

The filter means **30** is made of a porous material capable of drawing liquid within its structure by capillary action. The pores of the filter **30** should be sufficiently small to effect a filter separation of an insolubilized component within the liquid from a solubilized component. The filter may be composed of materials such as glass fiber filter paper, nitrocellulose, plastic, synthetic polymer, cellulose, cellulose acetate, polytetrafluoroethylene, polyethylene, polypropylene, polyvinylidene fluoride, or any other material formable into a filter having the qualities and characteristics as described above. In many applications it is desirable to use a material which is inert and chemically nonreactive with the analytes and washing solvents with which the test device is to be used. It has been found that a filter means, which comprises a microporous membrane having substantially uniform pores between 25 nanometers and 25 micrometers, has the characteristics described and is useful in performing immunoassay testing procedures for which this device is particularly useful. Examples of filters which may be used include filter paper known as WHATMAN GF/D and filter disc made by MICRO FILTRATION SYSTEMS of borosilicate glass known as GD-120 standard filter discs.

The apparatus of the present invention is beneficial where chemical reactions (typically the immunochemical reactions) occur external to the device and the final reactants fed into the filter means for separation of the unreacted elements therefrom. In external reactions, improved accuracy of component addition is possible. It is generally accepted that a longer incubation period can afford more complete reaction and binding of the reactants, thereby increasing the sensitivity of the assay systems that conduct the reactions solely within the filter means frequently are limited in the length of the incubation period. The filter can dry out during long incubation periods with such systems decreasing the sensitivity of the assay. In the present invention, because the reactions can take place externally to the device, much greater control and flexibility are obtained over the incubation period, greatly improving the overall sensitivity and specificity of the assay. In such instances the diagnostic device is used primarily as a separation device to separate soluble components from insoluble components within the liquid specimen poured into the input **20** of the inventive test device **10**. Thus, where it is desirable to perform numerous and varied assays without having a specific device for each specific assay, the inventive test device is useful. Such a nonspecific device may be composed of inert materials and therefore may be stored for an indefinite period of time and without refrigeration. Moreover, such nonspecific devices can be produced using mass production techniques at substantial cost savings.

The device **10** may also be used for specific immunochemical assays by "prespotting" the reaction zone with an analyte specific reactant. Prespotting is a term used to indicate that in a localized region **36** of filter means **30**, such as within reaction zone **32** only, a specific analyte reactant may be immobilized on the internal surfaces of the filter material. These internal surfaces define the interstices within the structure of the filter material. In prespotting, the reaction zone **32** of the device is prepared for direct use of a test specimen often without preparatory additions to the test device. For example, the manufacturer of the device could place in the filter reaction zone a binding protein to which an antibody is bound, which antibody is immunologically reactive with a specific antigen. Thus, a specimen being tested for the specific antigen would be poured into the test device inlet, flow through the discharge aperture and would be discharged onto the upper surface **37** of reaction zone **32** of filter **30**. The solution would be pulled by a wick through the reaction zone **32** which has been prespotted at **36**. After a sufficient incubation time, a washing solution would be added to the device and is again wicked through the reaction zone, washing unreacted components of the specimen outward into the peripheral zone, and into the absorbent means, thus stopping the immunological reaction. If the specific antigen is present in the specimen, it binds to the antigen's specific antibody which itself is already immobilized within the filter and would remain in the reaction zone after the washing step. The unbound antigen and other material within the solution are effectively washed away from the reaction zone and into the absorbent means **40**. Finally, an antibody labeled with a detectable enzyme, such as an enzyme which generates a particular color of light, is poured through the test device and binds to the bound antigen. A washing solution is again added after a desired incubation period to remove all unbound enzyme labeled antibodies. Then the reaction zone is viewed through the viewing port **54** to determine if the color produced by the enzyme is present and, if so, in what amounts. The presence of the enzyme indirectly indicates that the antigen was indeed within the sample specimen. The absence of the enzyme indicates no antigen was present.

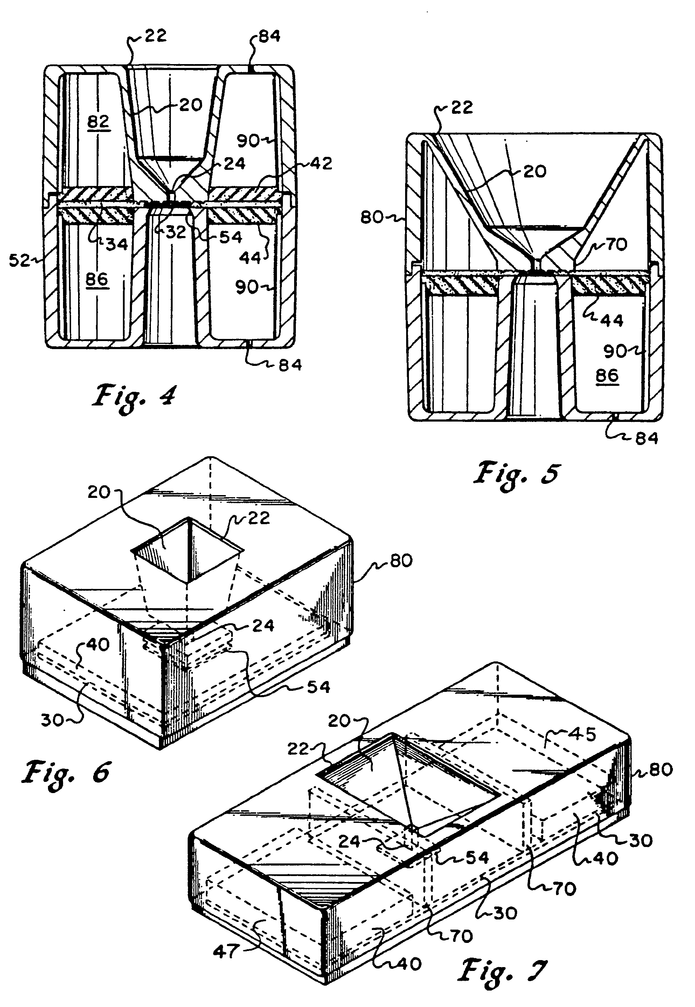

As shown in **Fig. 2**, the preferred embodiment of the test device is formed substantially symmetrically around a vertical axis. The absorbent means **40** is a skirt **40** of absorbent material which entirely surrounds the discharge aperture **24** of the input funnel **20**. The absorbent material may be any suitable material such as hydrophilic polymers, particulate absorbents, glass fiber, cotton fiber, cellulose fiber, wood pulp, or sponge material. In the preferred embodiment a compressed sponge material is used which expands upon absorbing liquid. In other embodiments of this invention absorbent means **40** may be positioned partially encompassing the periphery of reaction zone **32**, such as **Figs. 7, 8,** and **9**.

The filter means **30** is formed in a flat circular disc shape. The closure means **52** is of a size and shape corresponding to the filter means **30**. This configuration promotes uniform flow outwardly from the point at which liquid is discharged from discharge aperture **24** and onto reaction zone **32**. The uniform flow outwardly through filter **30** as provided by this inventive test device is advantageous over other test devices for several reasons. It is desirable to have all of the specimen pass through a localized zone of the filter material, and to have relatively large quantities of liquid pass through the localized zone. This is accomplished by making the receiving inlet **22** larger than the discharge aperture **24** so that large quantities of liquid are funneled for localized discharge onto the reaction zone **32**. The outward flow permits a large area of absorbent material to be in contact with the filter so that large quantities of liquid can pass through the filter means while maintaining a small reaction zone. Further, to promote the passage of the liquid through the reaction zone rather than merely spreading across the surface of the filter, a substantially liquid impermeable shield **70** contacts the surface of the filter means **30** separating the discharge aperture **24** from the absorbent means **40**.

It is important that fluid applied to the reaction zone area pass into the filter means and flow outwardly into the absorbent means. Flow of fluid across the top of the filter means must be impeded so as to ensure the chromatographic separation of the bound from unbound materials via capillary action. As shown in **Fig. 2**, the shield means **70** is provided by abutting the bottom end **26** of the liquid input means **20** against the filter means **30**. The shielding action is promoted by having at least one serration **72** which contacts the upper surface of the filter means and compresses it slightly to provide an impediment to surface flow so that the liquid is directed into and must pass through at least a portion of the reaction zone **32** structure via the interstices within the reaction zone of the filter means **30**. To promote shielding with minimum localized compression of filter means **30**, multiple serrations are used in the preferred embodiment as shown in the drawings. Alternatively, the shield means **70** can be permanently affixed to the filter means, ensuring the separation of the discharge aperture from the absorbent means.

A unique aspect of the present invention is the ability to read and illuminate from either the top or bottom surface. Other systems are limited to reading the reaction from either only the top or only the bottom surface and typically require illumination from the same side as it is being read ; these include filter discs, dipsticks, tabs containing filter materials, devices where the illumination opening and the reading opening are the same, etc. The capability of reading from either surface enhances the flexibility and adaptability of the present invention to different reacton systems and analyzing instruments. A beneficial aspect of the inventive test device is that the reaction zone can be viewed from the bottom. As shown in **Fig. 2**, closure means **52** has formed therein a viewing port which is held in alignment with the reaction zone of the filter means. In the embodiment shown the filter means **30** is a flat circular disc shape, the absorbent means **40** is in the shape of an annular ring, and the reaction zone is a circular disc shape. The viewing port **54** permits easy viewing of the bottom face **39** of the reaction zone **32** without any interference or obstruction caused by either the input means **20** itself, or by debris and particulate or colored matter which may have been present in the liquid specimen. It is especially advantageous to have a device which permits bottom reading where the liquid specimen comprises bodily fluids such as blood, urine, feces, mucus, or other specimens which may be colored or in which contaminants may be present. Such contaminants include colored red blood cells, dead cellular materials from mucosal specimens, various colored debris, and food particles from feces, crystalline or other precipitates from urine, etc. The prior art devices require that the samples be cleansed of particulate matter as by centrifuge devices prior to testing. Viewing port 54 is made sufficiently large to allow light to enter so that an accurate reaction reading can be made. The test device is versatile and the reaction can also be read by illuminating the top surface and reading from same through liquid input means 20 where such a reading is desirable, as where the sample specimen is relatively free of particulate matter or as where the particulate matter entrapped near the upper face of the reaction is of particular importance to the test. Moreover, with the present test device it is possible to illuminate from the top surface and read the bottom surface, or, illuminate the bottom surface and read from the top. This unique ability permits convenient instrument reading of samples by measuring the absorbance of light by material present in the filter. An assay particularly well suited for this is an enzyme substrate system whereby the density of substrate as related to the presence of analyte is measured by the increased absorbance of light passing through the filter. Since a reading can be made from either surface, the advantage is in its adaptability to different instruments.

The liquid impermeable shield 70 can be designed to enhance the readability of the reaction by having the bottom surface of the shield be either light absorbency, light reflecting, or light transmitting, depending on the desired method of reading the reaction. For example, when it is desired to both illuminate and read from the bottom surface, a reflection surface at which light received through the filter will reflect off the sample and exit the bottom surface, thereby enhancing readability of the reaction. When it is desirable to eliminate reflection, reading through the filter by illuminating the opposite surface can be enhanced by designing the shield to be light transmitting ; in this manner the instrument reading can be improved.

As can be seen with reference to **Fig. 2**, enclosure means 52 provides a flat surface 56 to support the substantially flat bottom face 39 of the peripheral zone 34 of filter means 30. Also alignment means 58, which may be a circular ridge 58 integrally formed on holding means 52 has an inside diameter corresponding to the outside diameter 38 of filter disc 30 and an outside diameter corresponding to the inside diameter of lip 88 of casing 80. Likewise, absorbent means 40 has an outside diameter 48 corresponding to the outside diameter 38 of filter means 30 and the inside diameter of alignment means 58. Thus, all the component parts of the invention are fitted together and thereby held in alignment. In particular, the reaction zone 32 is held aligned with discharge aperture 24 for receiving liquid therefrom. Alignment of the reaction zone and the discharge aperture is crucial for accurate reproducible results. The funneling means consistently delivers fluid precisely to the same position on the filter via the discharge aperture, thereby eliminating random positioning errors by manual manipulations or mechanical means. The accurate positioning of fluids onto substantially the center of the reaction zone affords greater accuracy. Where the reaction occurs external to the device, all fluids added to the device are guaranteed to be applied to the same point because of this alignment. Furthermore, where the filter is prespotted with a component, such as an antibody, accuracy is ensured by the correct addition of antigen and wash fluids precisely to the prespotted area. Other devices introduce user error by not having a fluid delivery system funneling means aligned with the reaction zone of each device. Some other systems have a large area of filter containing a small reaction zone whereby a user must approximate where the colorless prespotted component is located. If not located precisely in the center, incomplete binding or washing can occur, reducing the overall accuracy and sensitivity of the assay.

The upper face 37 of the filter 30 will trap colored or particulate matter contained within the specimen and prevent such insoluble matter from reaching the bottom face 39 of the reaction zone 32. Only the soluble material will diffuse outwardly through the filter and down to the bottom face 39. The reading port 54 as described above is held in alignment with the reaction zone to afford a reaction reading signal that is free from false coloration or extraneous matter.

To provide maximum separation of bound from unbound label components and thereby reduce background noise during observation of the reaction, the invention provides for outward diffusion of liquid applied to the filter toward the absorbent material 40. The filter material 30 serves not only as a means for trapping and immobilizing particulate matter and reaction components, but also as a means for liquid transfer from the point of application to the absorbent material 40 so as to effect a filter separation. Diffusion of the material outward from the center point of application rather than directly down through the filter provides a more effective separation, particularly during the washing step where the unbound components are to be removed from the bound components. Properly performed in the present inventive apparatus, an assay procedure will leave a concentrated spot of bound label in the reaction zone 32 of the filter material and immediately surrounding the reaction zone 32 will be clear peripheral zone 34 containing negligible signal generating material, and the unbound label will be washed away from the observation area of the reaction zone.

To maximize the contrasting zones, relatively large quantities of washing solution are required. To promote effective transfer of the relatively large quantity of liquid from the peripheral zone **34** to the absorbent means **40**, the annular ring **40** of absorbent material is held in intimate contact with a portion of peripheral zone **34**. A large transfer area can be accomplished by forming the annular absorbent ring in the shape of a hollow cylinder of substantially uniform thickness having a flat base **49** which is held in intimate contact with the upper face **37** of the peripheral zone **34** of the filter means **30**. Continuous and complete contact is promoted by forming at least one, and preferably more than one, evenly-spaced projections **90** on the internal surface of cavity **82**. Frictional contact between absorbent means **40** and projections **90** acts to restrict the movement of absorbent means **40** upward into the hollow cavity **82**. Thus, it is held in intimate contact with the filter surface for direct transfer of liquid therefrom.

Where the hollow cylinder absorbent material is compressed sponge material, it will be relatively rigid in its dry state and will become more flexible upon absorbing liquid and expanding. The additional flexibility permits the absorbent material to expand deforming slightly to accommodate projections **90** as it expands into hollow cavity **82**. The air displaced thereby is permitted to escape through vent **84**. As shown in **Fig. 1** by dashed lines **92** and directional arrows **94**, the compressed absorbent material may expand several times its original thickness upon absorbing the substantial amount of liquid used in the testing device.

The absorbent material makes possible the use of a very large volume of wash fluid. Generally, a more effective separation is obtained when using large wash volumes. In other devices not containing absorbent material, the amount of wash solution that can be used is limited by the absorbent characteristics of the filter material, typically much less than an absorbent material such as a sponge or compressed wood pulp material.

While the invention thus far has been described with respect to a preferred embodiment, reference to **Figs. 4, 5, 6, 7, 8, 9,** and **10** shows additional alternative embodiments.

Fig. 4 shows a test device having absorbent material in contact with the upper and the lower peripheral faces of filter means **40**.

Fig. 5 shows an alternative embodiment in which absorbent means **40** is in contact with only the lower face of filter means **40**.

Fig. 6 shows a rectangular embodiment with rectangular shaped filter means **3** and with a rectangular absorbent skirt **40** surrounding discharge aperture **24**.

Fig. 7 shows an embodiment in which a filter means **30** is rectangular and the absorbent means **40** comprises two separate absorbent means **45** and **47** in contact with portions of peripheral zone **34** on either side of the reaction zone **32**.

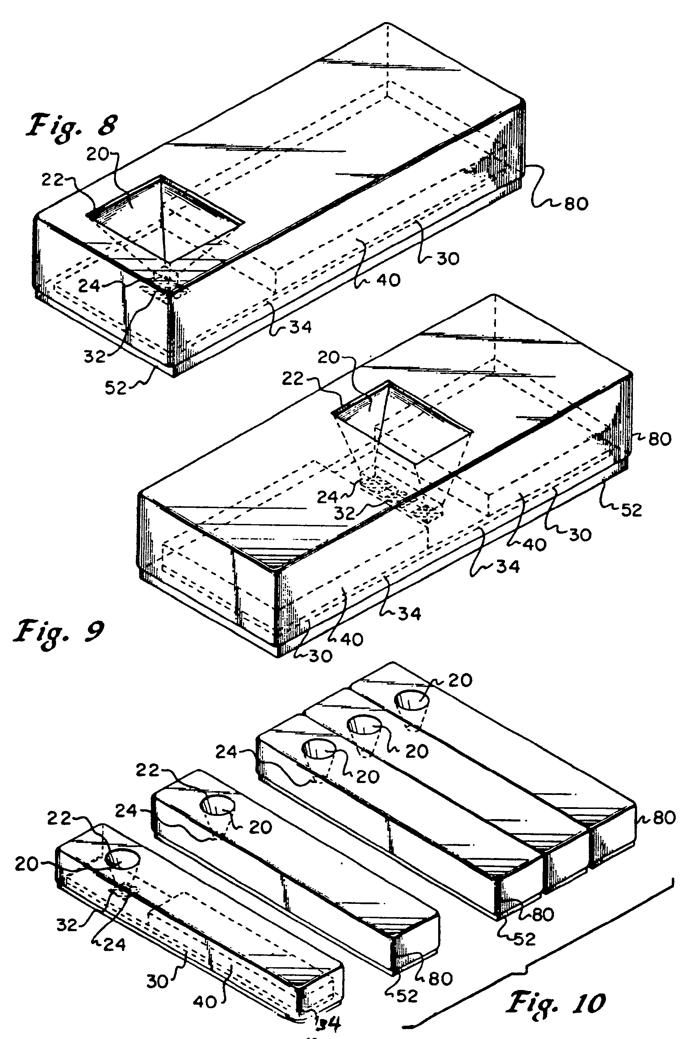

Fig. 8 shows a rectangular embodiment similar to Fig. 6 but has liquid input means **20** positioned at one end of the structure and absorbent means in contact with one edge of peripheral zone **34**.

Fig. 9 shows a rectangular embodiment in which liquid input means **20** is shaped as a trough containing a plurality of discharge apertures **24** in contact with filter means **40**. A plurality of discrete reaction zones **32** are thereby created.

Fig. 10 shows an embodiment whereby a plurality of individual devices have been joined together to form a multipurpose, multiparametric system. A user can randomly select any desired units and snap or fasten them together.

One common inventive aspect of each of these alternative embodiments is that the filter means **30** is positioned below the input means **20** with at least one reaction zone **32** for receiving liquid from the input means **20** and having at least one peripheral zone **34** associated with the reaction zone **32**. The absorbent means **40** is associated with only the peripheral zone **34** of the filter means **30** and the retainer means **50** holds the filter means in position below the liquid input means **20** such that the reaction zone receives liquid therefrom.

Further inventive aspects are that there is an enclosure means **52** corresponding in size and shape to the filter means **30** for holding the reaction zone **32** in alignment with the discharge aperture **24** and the enclosure means **52** has a port **54** in alignment with the reaction zone **32** for viewing the reaction zone **32** from below. Another aspect is that the receiving inlet **22** is larger than the discharge aperture so that liquid poured into the inlet **22** is funneled for localized discharge onto the reaction zone **32**.

A unique aspect of the embodiment of **Figs. 7, 8,** and **9** is that they lend themselves conveniently to multiple assay use. In such an application, each test device could contain a fastening means for removably or permanently attaching two or more devices together, side by side. This way a user could select from a stock of devices any one or several which are useful for the particular test to be done. Where the device has a component prespotted on the filter, for example, **Fig. 7**, a user could randomly select a device for each test to be performed in a series for a given patient, snap them together to form a unified set of devices, and run all the assays together. This use can be considered a multiparametric application because of the plurality of tests that can be performed with a number of devices.

Another novel aspect of this invention is shown by the embodiment in **Fig. 9**, wherein there are a plurality of reaction zones contained in a single device. As such, the device becomes more versatile where a plurality of reactions with the same components are desired to measure consistency of results. Moreover, where filter means 30 is prespotted, a plurality of different components, for example, antibodies, could be contained in a single device. Therefore, a set of different multiparametric reactions could be carried out simultaneously using a common input means 22 to deliver sample and reactants to different discrete reaction zones.

It will be evident from the foregoing that the devices of the present invention can be inexpensively made and thus disposed after being used for one test; or, if desired, the device 10 can be opened by separating casing means 80 and enclosure means 52 and replacing used filter means 30 and absorbent means 40 with new counterparts and reassembling the device for a further test.

Also, the present device can be used with chromogenic assays such that the test results can be observed by the eye of the user of the device or the device can be used in conjunction with an automated reader such as a colorimeter for determination of the results.

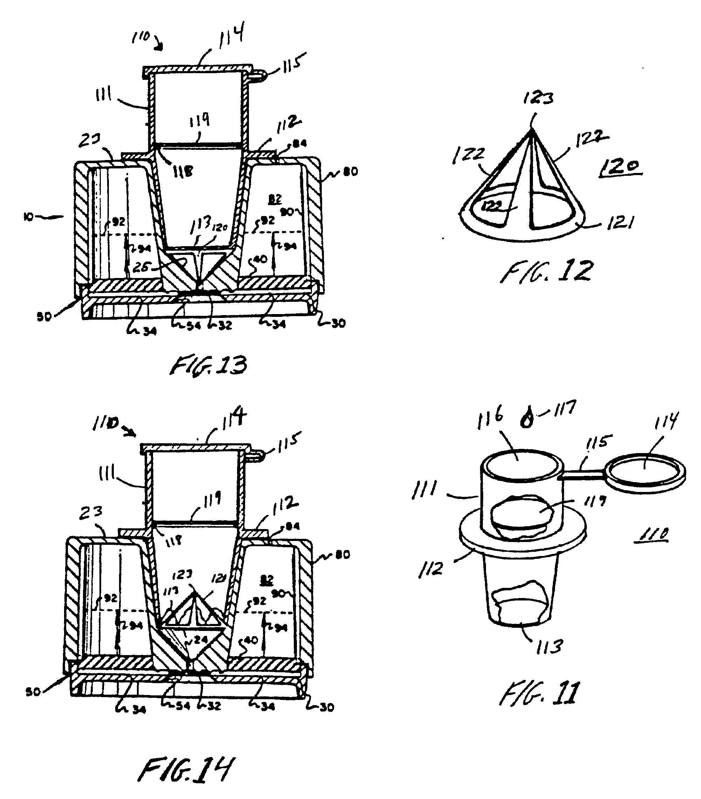

As shown in **Fig. 11** sample container **110** comprises a substantially cylindrical hollow tube means **111** having a liquid input means **116** at the top end and a substantially flat liquid impermeable or semi-permeable closure means **113** at the bottom end. Surrounding and permanently affixed to or integrated with the outside of tube means **111** is a substantially flat radially extending flange support means **112** for supporting the sample container **110** when positioned within the open end of the test device **10** as shown in **Fig. 13**. Materials such as fluid **117** can enter the sample container **110** through liquid input means **116** and react within the container. In an alternative embodiment it may be desirable to axially affix to or otherwise be supported by a circumferential ledge **118** on the inner wall of tube **111** a semi-permeable filter **119** for prefiltering the reaction components, such as where a feces specimen containing undesirable solid particles (e.g., food or red blood cells) is to be assayed. A removable top closure means **114** may be employed to cover top end **116** such as where the contents of sample container **110** are to be shaken, mixed, vortexed or otherwise agitated. Closure means **114** may be a nonaffixed cap or cover or can be attached to tube means **111** in proximity to liquid input end **116** by a flexible joint means **115**. Cover means **114** will provide a tight seal over tube **111** yet be removable by the user, if desired.

Sample container means **110** is sized as to removably fit within the liquid input means **20** (shown in **Figs. 1, 13, 15,** and **17**) of test device **10** by supporting the underside of flange **112** on the upper surface **23** surrounding receiving inlet **22**. This permits the stable positioning of sample container means **110**.

Puncture means **120** (shown in detail in **Fig. 12**) is suitable for use where the bottom end **113** of container **110** is a thin frangible surface or membrane covering tube **111**. Puncture means **120** comprises a base **121** with at least one arm **122** forming a relatively sharp point **123** capable of piercing the bottom surface **113**. Where several arms **122** are used, they join together in a conical shape to form a single sharp point **123**.

As can be seen in **Fig. 13**, puncture means **120** is positioned in conical portion **25** in the lower portion of liquid input means **20** with the point downwardly. The container **110** can then be placed on top thereof for storage purposes without the bottom frangible portion **113** being ruptured. This requires of course that the puncture means **120** be formed in a substantially conical shape which conforms to conical shape **25** in the lower portion of the liquid input means **20**. However, this configuration is advantageous inasmuch as it allows all of the components of the test device to be kept together for shipment and/or storage purposes.

When it is desired to utilize the test device **10** in performing a test, the puncture means **120** is removed and replaced within the lower portion of the liquid input means **20** with the point **123** facing upward toward the frangible bottom end **113** of container **110**.

As can be seen in **Fig. 14**, as pressure is applied downwardly on container **110**, points **123** are forced into intimate contact with bottom end **113** of container **110** piercing it, and thereby enabling the contents of container **110** to exit bottom end **113** and into test device **10** to be channeled by discharge aperture **24** onto reaction zone **32** of the filter **30**. Various embodiments of puncturing means **120** are possible such as, but not limited to, an array of spikes or protrusions supported by base **121** or an inverted hollow conical shape with the pointed end directed upwards and the surface of the conical shape perforated with at least one perforation to permit the passage of fluid therethrough.

The sample container **110** has a number of different functions which act to enhance the versatility and efficiency of the test device. By adding the sample and reactants to the container **110** without the filter means **119** therein, it becomes a reaction vessel with the capability of being agitated to facilitate reaction kinetics, as well as permitting extended incubation. In circumstances where a filter would dry out over a relatively long incubation period where the materials are reacting on or within the filter, a means for "off-line" incubation presents significant benefits. Control and flexibility of reaction times is increased by using such a sample container **110**. It can also be used as a specimen extraction processing container by enabling the user to pretreat a specimen prior to introduction to the reaction zone.

Semi-permeable membrane or filter **119** can be employed as a pre-filter means in container **110** whereby a specimen containing particulate matter may be pretreated to reduce the possibility of clogging reaction zone 32 of filter 30 with undesirable material. The membrane 119 can also be pre-impregnated or otherwise have immobilized on or within its pores reagents useful in a particular assay format or interest, so that materials added to the sample container 110 would come in intimate contact with the immobilized reagent while passing through membrane 119. Alternatively, reagents can be insolubilized, such as by lyophilization, on the inner walls of the container 110 which would become resolubilized upon addition of fluids to the sample container 110. Either method would save the user time by reducing the number of stages in the assay procedure. Spillage is also reduced by having fewer reagents to manipulate.

While a liquid input means that is integrally formed with device 10 as shown in **Fig. 1, Fig. 13,** and **Fig. 14** provides maximum compactness of form, as well as other important advantages, a removable liquid input means 130 as shown in **Fig. 15** presents certain improvements and advantages over the prior art. Input means 20 in **Fig. 15** still functions as a funnel means to concentrate liquids (i.e., reagents, specimen, and the like) onto the reaction zone 32 through discharge aperture 24. In order to achieve maximum efficiency of component separation on or within the filter, it is important that the liquid discharge aperture 24 be in contact with filter means 30. As previously described in relation to **Fig. 1,** at least one serration 72 contacts the upper surface of the filter means 30 compressing it slightly to provide an impediment to surface flow of liquid. This feature is retained in the present invention as shown in **Fig. 15** wherein the liquid barrier 70 can have at least one, and preferably multiple, serrations 72 designed and formed on it. The slight compression may occur sufficiently by simply having the container 110 resting in cap 130 in the test device 10 in a manner similar to that shown in **Fig. 7,** whereby its own weight provides the pressure required. Preferably, however, cap 130 is detachably affixed to test device 10 by any suitable means such as, but not limited to, screw threads 132 or may utilize other known methods of attachment such as snap-fit, slot-fit, groove, pressure fit, breakway, and the like. The only requirements include covenient removal of the cap or input means 130, and secure and consistent positioning of discharge aperture 24 in contact with reaction zone 32 while maintaining the slight compression of the filter means 30 by at least one serration 72. Many possible affixing means are possible and are readily adaptable to plastic molding and manufacturing techniques.

With a removable cap 130 having input means 20, a greater portion of the area surrounding reaction zone 32 may be made visible by varying the size of discharge aperture 24 as indicated by dashed line 134. This feature enables a user to compare any positive reaction product present in the reaction zone 32 to the area immediately surrounding reaction zone 32

which contains no reaction product. In the case of instrument measurement, a lens could be positioned closer to the filter means 30 than otherwise possible when the reaction zone 32 is receiving liquid.

By having a removable liquid input means or cap 130, the utility of using differently sized discharge apertures 24 becomes significant. Different assay procedures and different analytes typically require some optimization of the rate at which reagents and sample are added to the reaction area. A discharge aperture of a given diameter possesses particular flow dynamics for a given liquid density and quantity. It is desirable to control this flow rate in a test device such as the one disclosed, and, having the option to use one of several different liquid input means or caps 130, each possessing a particular aperture diameter, gives the user much greater flexibility in optimizing the performance of an assay.

Additionally, a removable liquid input means or cap 130 allows for the use of one aperture size for applying liquid to the filter means 30 and, when cap 130 is removed, a cap 130 with a different aperture size can be used transmitting light to the filter means 30 for reading the reaction products ; therefore, greater sensitivity of measurement may be obtained.

A filter membrane 136 can be placed within the liquid input means or cap 130, if desired, to serve as a pre-filter means before addition of the liquid material onto the reaction zone. In a manner similar to the pre-filter membrane 119 of the sample container 110 discussed previously, such a pre-filter means 136 can be removably or permanently associated with the liquid input means or cap 130 and may or may not have materials prespotted on or within the membrane. This feature would enable a user to pour in samples that may contain particulate or crystalline matter that might otherwise clog the reaction zone 32 of filter means 30.

As can be seen in **Fig. 16,** the substantially cylindrical body portion 138 has attached to it in the manner described previously in relation to **Fig. 1,** retainer portion 52 which holds filter 30 and absorbent means 40 in their proper relationship with respect to cylindrical body 138. On the upper end of body portion 138 are threads 140 which match with threads 132 on cap 130 shown in **Fig. 15.** Thus, cap 130 can be placed with liquid input means 20 extending into cylindrical body portion 138 and then cap 130 is rotated to mate threads 132 with 140 for attachment or may be attached in any other well known manner as set forth previously.

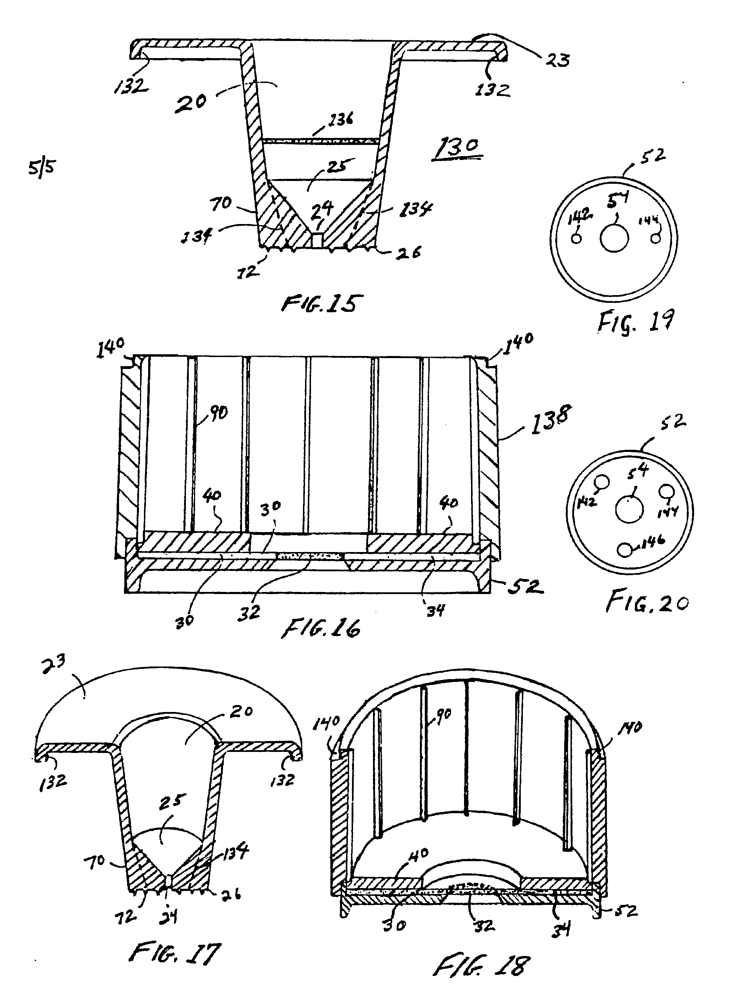

**Figs. 17** and **18** are cross-sectional isometric view of cap 130 and cylindrical body portion 138 shown in **Figs. 15** and **16. Fig. 17** is an embodiment which does not include the filter 136 shown in **Fig. 15** but it obviously could be included if desired. Device 10, reaction container 110, puncturing means 120, cap 130, cylindrical body portion 138, and retaining means **52** could be formed of any suitable inert material such as molded polystyrene, polyethylene or other plastic materials. The walls of container **10** may be either rigid or flexible. Further, as stated previously, cap **130** may be removably associated with cylindrical body portion **138** by detachably securing it in a well known manner such as by snap-fit, screw threads, pressure-fit, grooves, breakaway portions, or any other well known fastening means.

**Fig. 19** is a bottom view of the closure or retainer means **52** illustrating a modification thereof in which viewing ports **142** and **144** are formed in addition to the center viewing port **54**. This embodiment provides enhanced visual comparison of the reaction zone **54** and any color developed therein with a white negative background. Where color forms in the ports outside of the reaction zone, improper washing may have occurred. Without control ports **142** and **144**, even if no reaction products are present to cause a particular reaction color to be visible in port **54**, there may be, without proper washing, a reaction color caused by products other than the desired test product in the sample being tested. If that is the case, a false color may show up in center viewing port **54** indicating a positive test when in fact it is a negative test. By the use of additional control ports **142** and **144**, improper washing can be detected. If a negative reaction actually exists in center viewing port **54** and proper washing has occurred, then no color will exist in center viewing port **54** or spaced ports **142** and **144**. If a positive indication shows up in center viewing port **54** only because of improper washing, that same reaction indication will appear in spaced ports **142** and **144** thus indicating that there has been an improper washing. If there is a positive reaction in center viewing port **54** with proper washing, then no reaction will show in spaced ports **142** and **144**. Thus, a step of improper washing can be detected by the use of additional ports **142** and **144** so as to distinguish a false positive reaction in center viewing port **54** from a true positive reaction.

In addition, the embodiment shown in **Fig. 20** can be used where again first and second additional spaced ports **142** and **144** are used to detect improper washing as described in relationship to **Fig. 19**. In addition, however, in **Fig. 20**, a third spaced port **146** is added which is a control standard port. This location is treated in any conventional manner so as to produce a visibly colored reaction when substrate solution is added thereby acting as an internal control. This would indicate, regardless of the presence or concentration of analyte, that the proper assay procedures have been followed. For example, if a pregnancy test were to be conducted, the filter pad would have HCG immobilized at the location of third viewing port **146**. Addition of the enzyme-linked antibody and subsequently of the substrate will cause a color change (a positive reaction) as port **146** which in fact it should have if the proper immunoassay procedure has been followed. If then the same color reaction shows up at center viewing port **54** after the substrate solution is added, then the positive test is confirmed. If no color appears in center viewing port **54** but a color is present in third port **146**, a negative test is confirmed. In addition, of course, ports **142** and **144** would again remain clear with no reactions if proper washing has taken place. If improper washing has taken place, then, again, these ports **142** and **144** would show a reaction which would mean that the test would have to be redone.

Thus the embodiment shown in **Fig. 19** provides for a quick determination of whether or not proper washing has occurred while embodiment in **Fig. 20** not only provides the determination of proper washing but also provides a control standard to confirm whether or not the reaction indicated in port **54** is truly a positive or a negative test and whether the proper assay steps have been performed.

Thus, there has been disclosed an improved diagnostic device for analyte assay which includes a cylindrical body portion and a removable cap portion having a liquid input means that can include a variable diameter liquid discharge aperture. In addition, the cap portion may have a filter membrane within the liquid input portion thereof if desired or may be associated with a pre-mixing container that has a removable cap thereon, a filter internally thereof and a bottom portion which has a frangible membrane thereover for sealing the container. Thus, pre-mixing may occur in the container. A puncturing device is associated with the test device and may be placed in the lower portion of the liquid input means with the point downward for storage whereby the pre-mixing container may also be placed in the liquid means without the puncturing device rupturing the frangible lower seal thereof. When the device is ready to be used, the puncturing device can be removed and replaced in the liquid input means with the point facing upwardly and then after the liquids have been pre-mixed in the pre-mixing container, the container can be placed in the liquid input receiving portion of the removable cap where by the point of the puncturing device ruptures the frangible seal and allows the liquid in the pre-mixing container to be funneled by the discharge aperture onto the reaction zone of the filter. Thus, an improved diagnostic device and associated pre-mixing container have been disclosed.

The embodiments described and the alternative embodiments presented in the figures and in the detailed description of the drawings are intended as examples rather than as limitations. Thus, the description of the invention is not intended to limit the invention to the particular embodiments disclosed but it is intended to encompass all equivalents and subject matter within the scope of the invention as claimed in the claims which follow.

## Claims

1. A device (10) for testing for the presence of an analyte in a liquid, which comprises

an aperture (24) through which the liquid can be introduced ;

a filter (30) comprising a layer of porous material capable of drawing liquid through its structure by capillary action and which includes a reaction zone (32) which receives liquid through the aperture, and which functions to separate the analyte, if present, from the liquid ;

absorbent means (40) and means (50) for retaining the filter in position beneath the aperture ;

characterizd in that the filter includes a peripheral zone (34) and said reaction zone (32) which is adapted to be viewable associated with the peripheral zone, but not the reaction zone, of the filter, whereby liquid is drawn from the reaction zone to the peripheral zone.

2. A test device according to claim 1, adapted for downward liquid flow through the aperture on to the reaction zone, wherein the filter is substantially horizontally-oriented, with respect to liquid flow.

3. A test device according to claim 2, wherein the absorbent means comprises an absorbent skirt which abuts the lower face of the peripheral zone, entirely surrounding the reaction zone.

4. A test device according to claim 2, wherein the absorbent means comprises first and second absorbent skirts (45, 47) in contact with the upper face of first and second portions of the peripheral zone, such that neither absorbent skirt entirely surrounds the reaction zone.

5. A test device according to claim 3 or claim 4, wherein the absorbent means comprises an absorbent skirt which abuts the upper face of the peripheral zone, entirely surrounding the reaction zone.

6. A test device according to claim 5, wherein the absorbent skirt is in the form of a hollow cylinder coaxial with the aperture and whose base is in contact with the upper face of the peripheral zone of the filter.

7. A test device according to claim 6, wherein the filter is in the form of a circular disc whose perimeter corresponds to the perimeter of the hollow cylinder.

8. A test device according to any of Claims 3 to 7, which further comprises a hollow cavity (82) containing the absorbent skirt, and wherein the skirt comprises a compressed sponge material which, upon contact with liquid, absorbs the liquid and expands into the cavity.

9. A test device according to claim 8, which further comprises a vent (84) to the hollow cavity, to permit equalisation of air pressure within and outside the cavity, upon expansion of the sponge material.

10. A test device according to claim 8 or claim 9, which further comprises a projection (90) from the interior of the hollow cavity, having a size sufficient to restrict movement of the compressed sponge material and to hold the compressed and expanded sponge material in contact with the filter, while permitting expansion of the sponge material into the cavity with plastic deformation around the projection.

11. A test device according to any preceding claim, wherein the aperture is defined by liquid-impermeable material permanently affixed to the filter.

12. A test device according to any preceding claim, wherein the aperture is defined by liquid-impermeable material having a substantially flat surface carrying one or more circular serrations (72) which are in intimate contact with the filter, and surrounding the aperture.

13. A test device according to any preceding claim, wherein the filter comprises a micro-porous membrane having substantially uniform pores of a size between 0.025 and 25 μm, the pores defining integral surfaces in the membrane, and an analyte-specific reactant immobilised on the integral surfaces.

14. A test device according to any preceding claim, wherein the filter is composed of glass fibres.

15. A test device according to any preceding claim, wherein the device further comprises a liquid-receiving inlet (22) in connection with, and of a cross-section layer than, the aperture, so that liquid introduced into the inlet is funnelled for localised discharge on to the reaction zone.

16. A test device according to any preceding claim, wherein the retaining means comprises a base having a port (54) in alignment with the reaction zone, thereby allowing the reaction zone to be viewed from the side of the filter opposite to the aperture.

17. A test device according to claim 16, wherein the port is sufficiently small in size that liquid does not escape therethrough.

18. A test device according to claim 16 or claim 17, wherein the base additionally comprises one or more viewing ports (142) which are not in alignment with the aperture.

19. A test device according to claim 18, which comprises a non-aligned port (142) for viewing the peripheral zone.

20. A test device according to claim 18 or claim 19, which comprises a non-aligned port (146) for viewing a control standard associated with the filter.

21. A test device according to any of claims 15 to 20, which has a casing (80) including the liquid-receiving inlet and the base, the casing also defining the cavity, if present, specified in claim 8.

22. A test device according to claim 15 and any of claims 16 to 20, wherein the retaining means comprises a body portion including the aperture and the base, and wherein the liquid-receiving inlet is in the form of a removable cap.

23. A test device according to claim 22, wherein the liquid-receiving inlet is in the form of one or more removable caps providing a variety of inlet sizes.

24. A test device according to claim 22 or 23, which additionally comprises a reaction container (110) having a flange (112), for demountable secure location in the liquid-receiving inlet.

25. A test device according to claim 23, wherein the container comprises an upper opening (116) having a secure closure (114), optionally connected to the container by a flexible connector (115), and a lower opening sealed by a frangible membrane (113).

26. A test device according to claim 23, wherein the container has a filter membrane (119) mounted therewithin.

27. A test device according to claim 25 or claim 26, which additionally comprises means (120) for puncturing the frangible membrane on insertion of the container into the liquid-receiving inlet.

28. A test device according to claim 27, wherein the liquid-receiving inlet and the puncture means are generally conical, such that the puncture means can be stored point downwards in the inlet and used point (123) upwards, and wherein the puncture means has holes allowing liquid to pass therethrough.

29. A combined plurality of the test devices according to any preceding claim, permanently or removably joined together, suitable for multi-parametric use.

## Ansprüche

1. Vorrichtung (10) zum Testen des Vorhandenseins eines Analyten in einer Flüssigkeit, mit
einer Öffnung (24), durch welche die Flüssigkeit eingeführt werden kann ;
einem Filter (30), der eine Schicht aus porösem Material aufweist, die befähigt ist, durch Kapillarwirkung Flüssigkeit durch ihren Aufbau zu ziehen und die eine Reaktionszone (32) aufweist, welche die Flüssigkeit durch die Öffnung hindurch empfängt und die eine Trennung des Analyten, falls vorhanden, von der Flüssigkeit bewirkt ;
einem Absorptionsmittel (40) und einem Mittel (50) zum Zurückhalten des Filters in einer Position unterhalb der Öffnung ;
dadurch gekennzeichnet, daß der Filter eine Umfangszone (34) und eine Reaktionszone (32) umfaßt, die befähigt ist, betrachtet zu werden, und das Absorptionsmittel der Umfangszone, aber nicht der Reaktionszone des Filters zugeordnet ist, so daß Flüssigkeit aus der Reaktionszone zur Umfangszone gesaugt wird.

2. Testvorrichtung nach Anspruch 1, welche für eine Flüssigkeitsströmung abwärts durch die Öffnung auf die Reaktionszone eingerichtet ist, wobei das Filter bezüglich der Flüssigkeitsströmung im wesentlichen horizontal orientiert ist.

3. Testvorrichtung nach Anspruch 1, bei welcher das Absorptionsmittel einen absorbierenden Mantel aufweist, der an der Unterseite der Umfangszone anliegt und die Reaktionszone vollständig umgibt.

4. Testvorrichtung nach Anspruch 2, bei welcher das Absorptionsmittel einen ersten und einen zweiten absorbierenden Mantel (45, 47) aufweist, die mit der Oberseite eines ersten und eines zweiten Abschnittes der Umfangszone so in Berührung stehen, daß keiner der absorbierenden Mäntel die Reaktionszone vollständig umgibt.

5. Testvorrichtung nach Anspruch 3 oder 4, bei welcher das Absorptionsmittel einen absorbierenden Mantel aufweist, der an der Oberseite der Umfangszone anliegt und die Reaktionszone vollständig umgibt.

6. Testvorrichtung nach Anspruch 5, bei welcher der absorbierende Mantel in Form eines mit der Öffnung koaxialen Hohlzylinders ist, dessen Basis in Berührung mit der Oberseite der Umfangszone des Filters steht.

7. Testvorrichtung nach Anspruch 6, bei welcher das Filter in Form einer kreisförmigen Scheibe ist, deren Umfang dem Umfang des Hohlzylinders entspricht.

8. Testvorrichtung nach einem der Ansprüche 3 bis 7, welche weiters einen den absorbierenden Mantel enthaltenden Hohlraum (82) aufweist und bei welcher der Mantel ein komprimiertes Schwammaterial aufweist, welches bei Flüssigkeitskontakt die Flüssigkeit absorbiert und sich in den Hohlraum ausdehnt.

9. Testvorrichtung nach Anspruch 8, welche weiters eine Belüftungsöffnung (84) zu dem Hohlraum aufweist, um den Ausgleich des Luftdruckes innerhalb und außerhalb des Hohlraumes bei der Ausdehnung des Schwammaterials zu gestatten.

10. Testvorrichtung nach Anspruch 8 oder 9, welche weiters einen Vorsprung (90) an der Innenfläche des Hohlraumes aufweist, der eine ausreichende Größe besitzt, um die Bewegung des komprimierten Schwammaterials zu beschränken und das komprimierte und ausgedehnte Schwammaterial in Berührung mit dem Filter zu halten, während er die Ausdehnung des Schwammaterials in den Hohlraum unter plastischer Verformung um den Vorsprung herum gestattet.

11. Testvorrichtung nach einem der vorhergehenden Ansprüche, bei welcher die Öffnung durch ein flüssigkeitsundurchlässiges Material definiert ist, das permanent an dem Filter befestigt ist.

12. Testvorrichtung nach einem der vorhergehenden Ansprüche, bei welcher die Öffnung durch ein flüssigkeitsundurchlässiges Material definiert ist, das eine im wesentlichen flache Oberseite mit einer oder mehreren ringförmigen Zahnungen (72) besitzt, die in innigem Kontakt mit dem Filter sind und die Öffnung umgeben.

13. Testvorrichtung nach einem der vorhergehenden Ansprüche, bei welcher das Filter eine mikroporöse Membran mit im wesentlichen

gleichförmigen Poren einer Größe zwischen 0,025 und 25 μm aufweist, wobei die Poren integrale Oberflächen in der Membrane definieren, und das Filter einen analyten-spezifischen Reaktanten aufweist, der an den integralen Oberflächen immobilisiert ist.

14. Testvorrichtung nach einem der vorhergehenden Ansprüche, bei welcher das Filter aus Glasfasern zusammengesetzt ist.

15. Testvorrichtung nach einem der vorhergehenden Ansprüche, bei welcher die Vorrichtung weiters einen Flüssigkeitsaufnahmeeinlaß (22) in Verbindung mit der Öffnung und mit größerem Querschnitt als diese aufweist, so daß die in den Einlaß eingebrachte Flüssigkeit zur lokalisierten Abgabe auf die Reaktionszone befördert wird.

16. Testvorrichtung nach einem der vorhergehenden Ansprüche, bei welcher das Zurückhaltemittel eine Basis mit einer Sichtöffnung (54) in Ausrichtung mit der Reaktionszone aufweist, wodurch ein Betrachten der Reaktionszone von der der Öffnung gegenüberliegenden Seite des Filters ermöglicht wird.

17. Testvorrichtung nach Anspruch 16, bei welcher die Sichtöffnung ausreichend klein in der Größe ist, so daß die Flüssigkeit nicht durch diese entweicht.

18. Testvorrichtung nach Anspruch 16 oder 17, bei welcher die Basis zusätzlich eine oder mehrere Sichtöffnungen (142) aufweist, die nicht in Ausrichtung mit der Öffnung sind.

19. Testvorrichtung nach Anspruch 18, welche eine nicht-ausgerichtete Sichtöffnung (142) zum Betrachten der Umfangszone aufweist.

20. Testvorrichtung nach Anspruch 18 oder 19, welche eine nicht-ausgerichtete Sichtöffnung (176) zum Betrachten eines dem Filter zugeordneten Kontrollstandards aufweist.

21. Testvorrichtung nach einem der Ansprüche 15 bis 20, welche ein Gehäuse (80) besitzt, das den Flüssigkeitsaufnahmeeinlaß und die Basis beinhaltet, wobei das Gehäuse auch den in Anspruch 8 angegebenen Hohlraum, falls vorhanden, definiert.

22. Testvorrichtung nach Anspruch 15 und einem der Ansprüche 16 bis 20, bei welcher das Zurückhaltemittel einen die Öffnung und die Basis beinhaltenden Körperteil aufweist und bei welcher der Flüssigkeitsaufnahmeeinlaß in Form einer entfernbaren Kappe ist.

23. Testvorrichtung nach Anspruch 22, bei welcher der Flüssigkeitsaufnahmeeinlaß in Form einer oder mehrerer entfernbarer Kappen ist, die verschiedene Einlaßgrößen zur Verfügung stellen.

24. Testvorrichtung nach Anspruch 22 oder 23, welche zusätzlich einen Reaktionsbehälter (110) mit einem Flansch (112) zur entfernbaren sicheren Anordnung in dem Flüssigkeitsaufnahmeeinlaß aufweist.

25. Testvorrichtung nach Anspruch 23, bei welcher der Behälter eine obere Öffnung (116) mit einem sicheren Verschluß (114), der fakultativ durch einen flexiblen Verbinder (115) mit dem Behälter verbunden ist, und eine untere durch eine zerreißbare Membrane (113) abgedichtete Öffnung aufweist.

26. Testvorrichtung nach Anspruch 23, bei welcher der Behälter eine in ihm montierte Filtermembrane (119) aufweist.

27. Testvorrichtung nach Anspruch 25 oder 26, welche zusätzlich ein Mittel (120) zum Punktieren der zerreißbaren Membrane beim Einsetzen des Behälters in den Flüssigkeitsaufnahmeeinlaß aufweist.

28. Testvorrichtung nach Anspruch 27, bei welcher der Flüssigkeitsaufnahmeeinlaß und das Punktierungsmittel im wesentlichen derart konisch sind, daß das Punktierungsmittel mit der Spitze nach unten in dem Einlaß aufbewahrt und mit der Spitze (123) nach oben verwendet werden kann, und bei welcher das Punktierungsmittel Löcher aufweist, um das Durchtreten der Flüssigkeit zu gestatten.

29. Mehrere kombinierte Testvorrichtungen nach einem der vorhergehenden Ansprüche, permanent oder lösbar verbunden, für eine Multiparameter-Verwendung geeignet.

**Revendications**

1. Un dispositif (10) destiné à tester la présence d'une substance à analyser dans un liquide, lequel dispositif comprend :
   un orifice (24) à travers lequel le liquide peut être introduit ;
   un filtre (30) comportant une couche de matière poreuse capable de faire passer le liquide à travers sa structure par action capillaire et qui comporte une zone de réaction (32) qui reçoit le liquide à travers l'orifice, et qui fonctionne de manière à séparer la substance à analyser, s'il en est, du liquide ;
   des moyens d'absorption (40) et des moyens (50) de maintien du filtre en place, au-dessous de l'orifice;
   caractérisé en ce que le filtre comporte une zone périphérique (34) et une zone de réaction (32) qui est conçue pour être visible, les moyens d'absorption étant associés à la zone périphérique mais non à la zone de réaction du filtre, de sorte que le liquide est tiré de la zone de réaction vers la zone périphérique.

2. Dispositif de test selon la revendication 1, adapté à un écoulement de liquide vers le bas à travers l'orifice sur la zone de réaction, dans lequel le filtre est orienté de manière sensiblement horizontale par rapport à l'écoulement du liquide.

3. Dispositif de test selon la revendication 2, dans lequel les moyens d'absorption comportent une jupe absorbante qui vient buter sur la face inférieure de la zone périphérique, entourant complètement la zone de réaction.

4. Dispositif de test selon la revendication 2, dans lequel les moyens d'absorption comportent une première et une deuxième jupe absorbantes (45, 47), en contact avec la face supérieure d'une première et d'une deuxième partie de la zone périphérique, de telle manière qu'aucune des jupes absorbantes n'entoure complètement la zone de réaction.

5. Dispositif de test selon la revendication 3 ou la revendication 4, dans lequel les moyens d'absorption comportent une jupe absorbante qui vient buter sur la face supérieure de la zone périphérique, entourant complètement la zone de réaction.

6. Dispositif de test selon la revendication 5, dans lequel la jupe absorbante a la forme d'un cylindre creux de même axe que l'orifice et dont la base est en contact avec la face supérieure de la zone périphérique du filtre.

7. Dispositif de test selon la revendication 6, dans lequel le filtre a la forme d'un disque circulaire dont le périmètre correspond à celui du cylindre creux.

8. Dispositif de test selon l'une quelconque des revendications 3 à 7, qui comporte en outre une cavité creuse (82) contenant la jupe absorbante, et dans lequel la jupe comporte une matière spongieuse comprimée qui, au contact du liquide, absorbe ce liquide et gonfle dans la cavité.

9. Dispositif de test selon la revendication 8, qui comporte en outre un évent (84) dans la cavité creuse pour permettre l'égalisation de la pression de l'air à l'intérieur et à l'extérieur de la cavité, lors du gonflement de la matière spongieuse.

10. Dispositif de test selon la revendication 8 ou la revendication 9, qui comporte en outre une saillie (90) à l'intérieur de la cavité creuse, ayant une taille suffisante pour restreindre le mouvement de la matière spongieuse comprimée et pour maintenir la matière spongieuse gonflée et comprimée au contact du filtre, tout en autorisant la dilatation de la matière spongieuse dans la cavité, avec déformation plastique autour de la saillie.

11. Dispositif de test selon l'une quelconque des revendications précédentes, dans lequel l'orifice est délimité par un matériau imperméable au liquide fixé en permanence contre le filtre.

12. Dispositif de test selon l'une quelconque des revendications précédentes, dans lequel l'orifice est délimité par un matériau imperméable au liquide, ayant une surface sensiblement plate, portant une ou plusieurs dentelures circulaires (72) qui sont en contact très étroit avec le filtre et entourent l'orifice.

13. Dispositif de test selon l'une quelconque des revendications précédentes, dans lequel le filtre est constitué d'une membrane micro-poreuse ayant des pores de dimensions sensiblement uniformes, d'une taille comprise entre 0,025 et 25 µm, les pores définissant des surfaces faisant partie intégrante de la membrane, et sur lesquelles un réactif spécifique de la substance à analyser est immobilisé.

14. Dispositif de test selon l'une quelconque des revendications précédentes, dans lequel le filtre est constitué de fibres de verre.

15. Dispositif de test selon l'une quelconque des revendications précédentes, muni en outre d'une entrée (22) de réception de liquide reliée à l'orifice mais de section droite supérieure à celui-ci, de sorte que du liquide introduit dans l'entrée est guidé pour être amené de façon localisée dans la zone de réaction.

16. Dispositif de test selon l'une quelconque des revendications précédentes, dans lequel les moyens de maintien présentent une base munie d'un regard (54) aligné avec la zone de réaction, permettant ainsi d'observer la zone de réaction depuis le côté du filtre opposé à l'orifice.

17. Dispositif de test selon la revendication 16, dans lequel le regard est de taille suffisamment petite pour que le liquide ne puisse pas s'échapper à travers.

18. Dispositif de test selon la revendication 16 ou la revendication 17, dans lequel la base est munie en outre d'un ou plusieurs regards additionnels (142) qui ne sont pas alignés avec l'orifice.

19. Dispositif de test selon la revendication 18, qui comprend un regard non aligné (142) pour observer la zone périphérique.

20. Dispositif de test selon la revendication 18 ou la revendication 19, muni d'un regard (146) pour observer un standard de contrôle associé au filtre.

21. Dispositif de test selon l'une quelconque des revendications 15 à 20, ayant une enveloppe (80) comprenant l'entrée de réception du liquide et la base, l'enveloppe définissant en outre la cavité, si elle existe, spécifiée par la revendication 8.

22. Dispositif de test selon la revendication 15 et l'une quelconque des revendications 16 à 20, dans lequel les moyens de maintien comprennent un corps comportant l'orifice et la base, et dans lequel l'entrée de réception du liquide a la forme d'un bouchon amovible.

23. Dispositif de test selon la revendication 22, dans lequel l'entrée de réception du liquide a la forme d'un ou plusieurs bouchons amovibles fournissant différentes tailles d'orifices d'entrée.

24. Dispositif de test selon la revendication 22 ou la revendication 23, qui comporte en outre un récipient de réaction (110), muni d'une bride (112), pour pouvoir être positionné avec sûreté et de façon démontable dans l'entrée de réception de liquide.

25. Dispositif de test selon la revendication 23, dans lequel le récipient comporte une ouverture supérieure (116) avec fermeture de sécurité (114), qui peut être raccordée au récipient par une liaison flexible (115), et une ouverture inférieure fermée par une membrane fragile (113).

26. Dispositif de test selon la revendication 23, dans lequel le récipient possède une membrane filtrante (119) montée à l'intérieur.

27. Dispositif de test selon la revendication 25 ou la revendication 26, qui comporte en outre des moyens (120) de perforation de la membrane fragile lors de l'introduction du récipient dans l'entrée de réception de liquide.

28. Dispositif de test selon la revendication 27, dans lequel l'entrée de réception de liquide et les moyens de perforation ont une forme généralement conique, de telle sorte que les moyens de perforation peuvent être rangés avec la pointe tournée vers le bas dans l'entrée de liquide et avec la pointe (123), lorsqu'elle est utilisée, tournée vers le haut, et dans lequel les moyens de perforation ont des trous permettant l'écoulement du liquide à travers.

29. Une combinaison de plusieurs dispositifs de test selon l'une quelconque des revendications précédentes, réunis ensemble de façon permanente ou provisoire, apte à être utilisée pour plusieurs paramètres.

Fig. 1

Fig. 2

Fig. 3

Fig. 4

Fig. 5

Fig. 6

Fig. 7

Fig. 8

Fig. 9

Fig. 10

FIG. 13

FIG. 12

FIG. 14

FIG. 11

5/5

FIG.15

FIG. 19

FIG.16

FIG.20

FIG. 17

FIG. 18